# EUROPEAN PATENT SPECIFICATION

(11) **EP 3 649 920 B1**
(45) Date of publication and mention of the grant of the patent: **13.01.2021**
(21) Application number: 18205102.9
(22) Date of filing: 08.11.2018
(51) Int. Cl.: A61B 3/00, A61B 5/00, G02B 27/00, G02C 13/00

(54) **SYSTEM FOR DETECTING WHETHER A VISUAL BEHAVIOR MONITOR IS WORN BY THE USER**
SYSTEM ZUR ERFASSUNG, OB EIN VISUELLER VERHALTENSMONITOR VOM BENUTZER GEBRAUCHT WIRD
SYSTÈME DE DETECTION DE L'UTILISATION D'UN MONITEUR DE COMPORTEMENT VISUEL

(43) Date of publication of application: 13.05.2020
(73) Proprietor: Vivior AG, 8005 Zürich (CH)
(72) Inventor: Zakharov, Pavel, 8604 Volketswil (CH); Mrochen, Michael, 8183 Eglisau (CH)
(74) Representative: Frenkel, Matthias Alexander

(56) References cited:
- EP-A1- 3 228 237
- WO-A1-2013/050735
- WO-A1-2018/017997
- WO-A1-2018/184072

## Description

### Technical Field

The present invention generally relates to a visual behavior monitor wearing status detection system for detecting whether a visual behavior monitor, usable to monitor visual behavior of the user, is worn and/or used, in particular correctly used, by the user.

### Background

In the planning of vision correction procedures, it may be important to understand a patient's lifestyle in relation to vision, so-called visual behavior, in order to better customise the procedure. People have different visual needs and the ability to characterise and understand those is critical for identifying the best solution for a specific patient. For example, in a cataract surgery, a clouded natural lens may be replaced with a plastic implant. As a result, the operated eye loses the natural ability to accommodate and vision is fixed to the specific working distance. For the other distances, the patient is forced to rely on vision correction means, such as spectacles. By the selection of optical power of an intraocular lens, healthcare practitioner can match the working distance to the typical visual lifestyle of the patient and with this increase spectacles independence and overall comfort of the patient.

A patient's visual lifestyle can be characterised with a visual behavior monitor (VBM), which is typically a wearable device, which is fixed on the spectacles of the patient or directly on the patient's head, for example as an earpiece. A visual behavior monitor comprises sensors which monitor environmental factors, such as illumination conditions, orientation and motion of the VBM, as well as visual activity of the user, such as distances to objects in the visual field. From this information, the VBM can derive information about the user's activities relevant for a planned treatment.

In another application, the VBM is used to monitor the visual behavior of the user and intervene if an unhealthy behavior is detected. For example, detecting reading activity in low light conditions may trigger a warning to the user to improve illumination. In a similar way, spending too much time focused on mobile devices can also trigger an alarm. This is particularly useful for preventing or slowing progression of myopia among children.

A visual behavior monitor may comprise an activation button to start and stop measurements for visual behavior monitoring of a user. The user may hereby have to keep remembering to push the button to activate or deactivate visual behavior monitoring.

When the visual behavior monitor is put on and off spectacles regularly (for example, due to social considerations), it may happen that the user forgets to push the button for activation, resulting in visual behavior of the user not being monitored. This may have negative implications not only, but in particular with elderly patients, who may use the visual behavior monitor for a limited time, and elderly people may not be trained by anyone else regarding how to properly use the visual behavior monitor, which aggravates the problem. It will be understood, however, that the problem arising from implementing a manual control to start and stop measurements for visual behavior monitoring of a user is universal, and may also in particular detrimentally affect visual behavior monitoring of children.

An alternative to the manual activation control would be to have the visual behavior monitor permanently active and continuously collect the sensor measurement to monitor visual behavior, to then determine which data is relevant or not for subsequent data analysis. However, such an approach would result in increased battery power consumption and potentially a large amount of data, which may in fact be useless for visual behavior analysis, having to be analysed and/or stored in a memory.

There is therefore a need for improvements to visual behavior monitoring.

EP 3 228 237 A1 relates to a distance measuring system. The distance measuring system comprises a distance measuring sensor, a memory unit and a processing unit. The distance measuring sensor is adapted and arranged to measure viewing distances between a subject's eyes and one or more objects. The memory unit is adapted to store the measured viewing distances in a set of measured viewing distances. The processing unit is adapted to determine a statistical distribution of the measured viewing distances from the set of measured distances.

WO 2018/184072 A1 is directed to systems, methods and/or devices for monitor conditions of an end-user, conditions around and end-user. Systems, methods and/or devices for improving eye health, for example with respect to reducing the progression of myopia and/or axial eye growth of an end-user are disclosed. A described wearable apparatus comprises: one or more sensors and one or more actuators; the one or more sensors being arranged to collect data related to at least one condition of a user and send data for processing; the one or more actuators being arranged to receive actuating signals based at least in part on a result of the processing; wherein, the wearable apparatus is arranged to be positioned in proximity to the head of the user and upon receiving an actuating signal, the one or more actuators either trigger a variation of a property of the wearable apparatus or instruct the one or more actuators to not trigger the variation of the property of the wearable apparatus.

WO 2018/017997 A1 discloses a compliance monitor for wear on a head including a data logger with processor, and memory containing firmware; with sensors that may include one or more of magnetometers, accelerometers, gyroscopes, and temperature sensors. The firmware is adapted to process sensor readings to determine intervals where the compliance monitor is attached to a person's head, and in embodiments is adapted to scan for signatures indicating donning of the compliance monitor. The method of determining wear of a head-mounted device by a person includes determining differences between a magnetic field measured at the start of a time interval and the time interval, differences between a temperature reading at a sensor on a head side of the head-mounted device and on an ambient side of the head-mounted device, accelerations of the head-mounted device indicative of wear, and rotations of the head-mounted device indicative of wear. WO 2013/050735 A1 relates to spectacles, and to uses thereof for treating a wide range of eye and visual disorders, such as myopia, hyperopia, astigmatism or amblyopia etc.

### Summary

The invention is set out in claim 1. Preferred embodiments of the invention are outlined in the dependent claims.

According to a first aspect of the present disclosure, there is provided a visual behavior monitor wearing status detection system for detecting whether a visual behavior monitor, usable to monitor visual behavior of a user, is worn by the user, wherein the visual behavior monitor wearing status detection system comprises: sensors configured to obtain sensor data, and wherein the visual behavior monitor wearing status detection system is configured to process the sensor data to determine whether the visual behavior monitor is worn by the user.

The visual behavior monitor and/or the visual behavior monitor wearing status detection system may be fixable to, for example, spectacles and/or an ear (or in-ear), and/or on a headband and/or headgear and/or headset or another part of the user's head and/or another fixation.

The visual behavior monitor wearing status detection system as described herein may therefore use one or more clues (i.e. signals) from one or more sensors in order to automatically detect that the visual behavior monitor is worn by the user (or not). Examples of the visual behavior monitor wearing status detection system may be implemented when the visual behavior monitor is worn, for example, on the body and/or the head and/or spectacles.

Using the one or more sensors to obtain sensor data and processing the sensor data to determine whether the visual behavior monitor is worn by the user therefore allows avoiding an activation button from being implemented in order to pre-select data for visual behavior analysis. Instead, the sensor data may be processed and/or analysed to determine whether and/or when the visual behavior monitor is worn by the user, so that only visual behavior monitor data, obtained via the visual behavior monitor, corresponding to a time period when the visual behavior monitor is detected (the determination therefor being performed using the visual behavior monitor wearing status detection system) to be worn by the user is used in the visual behavior analysis. This may avoid a high burden of first filtering visual behavior monitor data obtained via the visual behavior monitor prior to analysing the visual behavior monitor data.

It is hereby to be noted that the determination as to whether the visual behavior monitor is worn by the user or not may relate to a single point in time and/or an extended period of time.

It is further to be noted that any references throughout the present disclosure as to the visual behavior monitor being worn (or not) may equally correspond to the visual behaviour monitor wearing status detection system being worn (or not) by the user, from which it may ultimately be determined as to whether the visual behaviour monitor is worn (or not). The visual behavior monitor may be attached to or integral to the visual behaviour monitor wearing status detection system.

Processing parts of the visual behavior monitor wearing status detection system may be provided in a cloud environment for enhanced processing capabilities. It will hereby be understood that cloud computing may refer to shared pools of configurable computer system resources which may be provisioned, for example, over the Internet.

In some examples of the visual behavior monitor wearing status detection system, the determining, by the visual behavior monitor wearing status detection system, whether the visual behavior monitor is worn by the user comprises automatically determining whether a predefined condition, derived by the visual behavior monitor wearing status detection system from the sensor data, is fulfilled. The predefined condition may hereby be dependent on the type of sensor or sensors used to obtain sensor data based on which it is determined as to whether the visual behavior monitor is worn by the user or not. The predefined condition may hereby be, for example, a threshold below or above which it is determined that the visual behavior monitor is worn by the user.

In some examples of the visual behavior monitor wearing status detection system, the determination whether the visual behavior monitor is worn by the user comprises calculating, by the visual behavior monitor wearing status detection system, a probability for the user to wear the visual behavior monitor and determining whether the probability/likelihood exceeds or falls below a threshold. This may be particularly advantageous as a binary result based on the sensor data used to determine whether the visual behavior monitor is worn by the user may not be immediately obtainable, but only after a calculation, based on the sensor data, of a probability for the user to wear the visual behavior monitor. A statistical determination as to whether the user wears the visual behavior monitor or not may then be made based on the probability exceeding or falling below the threshold probability.

Combination of multiple classifiers may be used to estimate a probability of a certain state of the visual behavior monitor (worn or not). For example, if each of the classifiers provides the probability of the visual behavior monitor being worn, the combined classification output can be based on the voting rule.

Alternatively, multiple signals, related to the status of being worn, may be used to train automatic classifier using machine learning techniques based on the training set of observations of the visual behavior monitor being worn and not being worn.

In some examples, the visual behavior monitor wearing status detection system further comprises a controller for controlling, based on the determination whether the visual behavior monitor is worn by the user, starting and/or stopping of the visual behavior monitoring of the user via the visual behavior monitor and/or activating and/or deactivating components of the visual behavior monitor. Such an automatic controlling for starting and/or stopping of the visual behavior monitoring of the user and/or activating and/or deactivating components of the visual behavior monitor may be particularly advantageous since battery consumption of the visual behavior monitor may be significantly reduced, and/or analysis of the visual behavior monitoring data obtained via the visual behavior monitor may be simplified since the amount of visual behavior monitoring data may be reduced to only that data which is obtained while the visual behavior monitor is worn by the user. An example of such controlling may be disabling or putting in low power consumption mode components which are not required for detection of the wearing state.

In some examples, determination of wearing status may be done retrospectively. For example, the processor may be configured not to trigger immediately suspending the measurements in the absence of, for example, motion, since a user and, correspondingly, the VBM can be relatively static for a short period of time. However, when the immobility time exceeds a threshold, the processor can conclude that the device has been taken off and left to rest. The processor can act accordingly by stopping further data acquisition and discarding the data back to the moment when the period without motion has started.

In some examples of the visual behavior monitor wearing status detection system, the one or more sensors comprise one or more orientation sensors for obtaining orientation data relating to an orientation of the visual behavior monitor wearing status detection system, and wherein the sensor data, processed to determine whether the visual behavior monitor is worn by the user, comprises said orientation data. This may be particularly advantageous when determining as to whether the user wears the visual behavior monitor, since a properly attached sensor may (at least in most instances) not be, for example, completely oriented upside down, as in the majority of situations, people maintain an upright vertical body and head position For example, rarely, the head and/or body can assume a horizontal position and, for example, very rarely an upside down orientation. On the other hand, if the visual behavior monitor is, for example, in a package, in a bag or on a desk, one can assume any possible orientation. For example, the orientation can be estimated in relation to Earth's gravitation or magnetic fields, and may be measured using, for example, an accelerometer or/and magnetometer. In some examples, a threshold of orientation may be introduced and measurements for visual behavior monitoring may be stopped if the visual behavior monitor / the visual behavior monitor wearing status detection system and/or the head of the user are too far from being upright.

In some examples, the visual behavior monitor wearing status detection system is configured to determine, from the orientation data, one or both of a pitch and a roll of the visual behavior monitor wearing status detection system, and wherein the determination whether the visual behavior monitor is worn by the user is based on the pitch and/or the roll of the visual behavior monitor wearing status detection system satisfying an orientation condition. This may allow for precise determination as to whether the visual behavior monitor is worn by the user based on one or both of the pitch and the roll of the visual behavior monitor wearing status detection system.

In some examples of the visual behavior monitor wearing status detection system, the one or more orientation sensors comprise a first accelerometer. Using an accelerometer (or multiple accelerometers), the orientation of the visual behavior monitor wearing status detection system, and hence the orientation of the visual behavior monitor which may be attached to or integral to the visual behavior monitor wearing status detection system, may be obtained.

In some examples of the visual behavior monitor wearing status detection system, the one or more sensors comprise one or more motion sensors for obtaining motion data relating to a type and/or an amount of motion of the visual behavior monitor wearing status detection system, and wherein the sensor data, processed to determine whether the visual behavior monitor is worn by the user, comprises said motion data. This may be particularly advantageous as, for example, a determination that the visual behavior monitor wearing status detection system, and hence the visual behavior monitoring have not moved for longer than a threshold period of time, it may be determined that the visual behavior monitor is not worn by the user. An amount of motion may hereby refer, for example, to the speed of motion and/or acceleration and/or frequency of those. Motion may be characterize by the changes of sensor signals determining position and/or orientation of the visual behavior monitor wearing status detection system. An amount of motion may be measured by statistical processing of such signals, for example, the energy of high-frequency changes of accelerometer signals.

Motion may also be characterized by the type, for example, by classifying the type of motion experienced by the VBM wearing status detection system, such as walking, running, being carried in the case, being transported in the car, etc.

Presence of specific type of motion or excessive motion may also indicate that the monitor is not being worn by the user. For example, excessive shaking may not be physically possible or extremely unlikely when the visual behavior monitor is worn by the user. And even if the monitor is in fact worn on the head of the user while experiencing excessive motion, the visual function may be compromised due to inability of the user to focus and the measurements of visual behavior monitor may be less relevant.

Thus the wearing state of the monitor may have a characteristic motion pattern, which is described by the type of motion and/or the amount of motion.

Regarding movement, it is to be noted that it will be understood that the body and/or the head may, at least rarely, be completely at rest for a prolonged time, whilst, if device may be left on the desk, it would be at rest for a certain period of time. Therefore, a threshold motion may be taken into account when determining as to whether the user wears the behavior monitor or not, and, in some examples, behavior monitoring measurements may be stopped if the amount of motion is under the threshold motion. Movement and/or motion may be measured with motion sensors, such as, but not limited to, one or more accelerometer, one or more gyroscopes, one or more magnetometers, etc.

In some examples, the visual behavior monitor wearing status detection system is configured to stop and/or prevent starting the visual behavior monitoring of the user via the visual behavior monitor and/or activate and/or deactivate components of the visual behavior monitor when a first time period, for which the amount of motion of the visual behavior monitor wearing status detection system falls outside of a predefined range (and/or, for example, has not identified motion characteristic to state of being worn), exceeds a first threshold time period. This may be advantageous since, for example, the visual behavior monitor wearing status detection system not having moved for longer than the first threshold time period may indicate, for example, that the user may have fallen asleep while wearing the visual behavior monitor, or the user may have taken off the visual behavior monitor and placed it on the stable surface. This may further allow for improved battery performance of the visual behavior monitor and/or only collect visual behavior monitoring data for a period of time or times during which the visual behavior monitor is worn by the user.

In some examples of the visual behavior monitor wearing status detection system, the one or more motion sensors comprise one or more of: an inertial measurement unit (inertial sensor), a gyroscope, a magnetic sensor, a magnetometer and a second accelerometer, in particular wherein the second accelerometer is integral to (i.e. the same as) the (first) accelerometer outlined above. The magnetic sensor may be, for example, a Hall-effect sensor which may, for example, be placed in the ear of the user, whereby the monitoring of visual behavior of the user may be activated with the presence of the magnetic sensor and/or magnetometer.

In some examples of the visual behavior monitor wearing status detection system, the one or more sensors comprise one or more distance sensors, i.e. viewing distance sensors for obtaining distance data relating to a distance between the visual behavior monitor wearing status detection system and an object of viewing activity, wherein the sensor data, processed to determine whether the visual behavior monitor is worn by the user, comprises said distance data, and wherein the visual behavior monitor wearing status detection system is configured to determine that the visual behavior monitor is not worn by the user when the distance is below a threshold distance, in particular when the distance is below the threshold distance for a second time period exceeding a second threshold time period. Additionally or alternatively, the one or more sensors comprise one or more proximity sensors for obtaining proximity data relating to a proximity of the visual behavior monitor wearing status detection system to a body part of the user (e.g. the user's head), wherein the sensor data, processed to determine whether the visual behavior monitor is worn by the user, comprises said proximity data, and wherein the visual behavior monitor wearing status detection system is configured to determine that the visual behavior monitor is worn by the user when the proximity is below a threshold proximity.

The visual behaviour monitor may use the distance sensor(s) in order to estimate a viewing distance. It may be co-directed with a user line of sight. The distance sensor(s) may be an optical, acoustical and/or an electromagnetic sensor(s), for example, a LASER, SONAR, LIDAR or RADAR. The distance measuring sensor may be a time-of-flight optical sensor. Functioning of the distance sensor may involve sending a probing wave or a pulse in the direction of interest and detecting the wave or pulse reflected by the object(s). By measuring temporal delay and/or frequency shift between emitted wave or pulse and detecting the reflected wave or pulse, the distance sensor may derive information about distance to the object(s) and/or relative speed of the object(s).

Regarding the one or more distance sensors, when the visual behavior monitor (and the visual behavior monitor wearing status detection system) is worn, the distance sensor(s) may measure a distance which may not be too small (for example not smaller than 10 cm), since under normal conditions, a user does not place an object so close, since physiologically it may not be possible and/or comfortable to perceive an object so close. On the other hand, when the visual behavior monitor/ visual behavior monitor wearing status detection system is, for example, in a storage/transport container, a case, a bag or a packet, shorter distances may be measured due to constriction of space in the container, etc. In some examples, by introducing a threshold for the measured distance, the status of being in the case and not being worn may be determined.

The proximity sensor(s) may be directed towards the user's body and used to identify presence of the user and/or status of being attached on the user's body. Proximity sensor may preferably be implemented in the non-contact manner, using optical, acoustical and/or an electromagnetic signals, and/or an electromagnetic sensor, for example a LASER, SONAR, LIDAR or RADAR. The proximity sensor may be a time-of-flight optical sensor. In another implementation, the proximity sensor may send the directed light beam towards the target and measures the amount of reflected light. Presence and amount of reflection serve as an indication of a target in proximity to the proximity sensor, and hence the VBM.

In relation to the proximity sensor(s), these may, in some examples, be directed towards an area which would be covered, for example, by the head of the user when the user wears, for example, spectacles to which the visual behavior monitor and the visual behavior monitor wearing status detection system may be attached. Therefore, in some examples, proximity and distance data may be obtained in order to determine as to whether the user wears the visual behavior monitor, whereby, in some examples, proximity data and distance data may relate to data obtained in relation to different monitoring directions via the proximity sensor(s) and the distance sensor(s), respectively.

In some examples of the visual behavior monitor wearing status detection system, the one or more sensors comprise one or more light sensors (for example one or more ambient light sensors) for obtaining light intensity data relating to a light intensity of ambient light impinging on the one or more light sensors, wherein the sensor data, processed to determine whether the visual behavior monitor is worn by the user, comprises said light intensity data, and wherein the visual behavior monitor wearing status detection system is configured to determine that the visual behavior monitor is not worn by the user when the light intensity is below a threshold light intensity, in particular wherein the visual behavior monitor wearing status detection system is configured to determine that the visual behavior monitor is not worn by the user when the light intensity is below the threshold light intensity for longer than a third threshold time period. When a user is using the visual behavior monitor / visual behavior monitor wearing status detection system, some illumination of these is expected, since otherwise a user may not see or perceive things and thus there may be no visual activity, so the measured ambient light level may need to be above a certain (relatively small) threshold, at least for a certain period of time. At the same time, when placed in, for example, a container, case or bag a relatively low light level may be measured which may be recognized with the one or more light sensors.

In some examples of the visual behavior monitor wearing status detection system, the one or more sensors comprise one or more attachment sensors for obtaining attachment data relating to an attachment of one or both of the visual behavior monitor wearing status detection system and the visual behavior monitor to spectacles and/or an ear or another part of the user's head and/or another fixation, wherein the sensor data, processed to determine whether the visual behavior monitor is worn by the user, comprises said attachment data, and wherein the visual behavior monitor wearing status detection system is configured to determine that the visual behavior monitor is worn by the user when it is determined that one or both of the visual behavior monitor wearing status detection system and the visual behavior monitor, respectively, are attached to the spectacles and/or the ear and/or the another part of the user's head and/or the fixation.

In one implementation, the VBM is attached to the spectacles using a mount. The mount may be supplied with magnets and the VBM recognizes the attachment with the sensor of magnetic field. Additionally or alternatively, the mount has exposed connected contact pads and the VBM wearing status detection system recognizes attachment by testing the connection between contact pads. Presence of connection indicates that the VBM is attached.

In another implementation, the VBM is fixed in the user's ear as an earpiece and is able to detect when the user is putting on spectacles.

In some examples, the one or more sensors may be attachment sensors for detection of the presence of, for example, spectacles or a mount/fixation.

The visual behavior monitor wearing status detection system comprises at least a first one and a second one of the sensors outlined above, wherein the visual behavior monitor wearing status detection system is configured to combine the sensor data obtained from the first and second sensors to statistically determine whether the user is correctly wearing the visual behavior monitor and/or is wearing the visual behavior monitor in a manner previously determined to be improper. For example, the motion and attachment determination may indicate that the visual behavior monitor is worn, but the distance sensor may show a short distance. This may be an indication of sensor obstruction while wearing the visual behavior monitor and the visual behavior monitor wearing status detection system (for example due to hair of the user), and thus a user may be warned accordingly, for example by a sound signal or an optical signal of a signal generator of the visual behavior monitor wearing status detection system. When the multiple sensors/clues/features are used to identify wearing state, it is also possible to detect sensors which are significantly different from other sensors (outliers). Such outliers can identify non-standard use (misuse) of the VBM and thus warn the user and/or record incorrect usage. For example, when the distance sensor is covered by the user's hear or headgear, it will report a short distance/obstructed view, which is not typical for the normal usage. In combination with other sensors, reporting state of being worn, this allows to identify that the VBM is in fact worn and the distance sensor is being obstructed and one may act correspondingly. In another example, the user might put on the device backwards. In this case, the proximity and ambient light sensors would report correct values. The distance sensor may also report plausible distances if it is not obstructed by hair. However, the orientation sensor would be reporting anomalous measurements, since the upwards orientation would prevail when the patient is tilting his/her head down, which can be recognized and reported back to the user.

In another example, some users have a habit of moving spectacles upwards on the top of the head. With motion, distance and proximity sensors reporting status of being worn, the orientation would report that the VBM has been consistently oriented upwards, which is not typical for the normal VBM usage. Upwards viewing orientations might be present in the data even with a correct usage of the VBM, for example, when the user is looking forwards, but consistent state during visual activity is not typical and can be detected with statistical methods by analysing sensors in combination. This would serve as an indicator of the VBM wrong usage and is going to be recorded and/or the user alarmed. Such kind of information can also be used as a characteristic of the visual behaviour, since it reports that the prescription of the spectacles is not optimal for the current activity of the user.

In another example, which may be combined with any one or more of the embodiments and example implementations as described herein, a combination of (one or more) distance and (one or more) orientation sensors can be used to determine a wrong attachment of the wearable (visual behavior monitor and/or visual behavior monitor wearing status detection system) on the spectacles and/or the head (and/or another body part). It may be typical that in the normal usage, most of the far viewing distances (e.g. above 2 meters) may occur when the user is looking directly forwards. It is thus possible with sufficient observation statistics to estimate an orientation of the wearable and compare it to the typical horizontal observation. In one implementation, the median of the pitch angle during far distance measurements can be used as an indicator of the attachment angle. For the correct attachment, one may expect a pitch angle median around zero level, corresponding to horizontal orientation. A median deviating from zero level may indicate improper wearable attachment or spectacles wearing with wearable or spectacles tilted upwards or downwards, depending on the sign of the pitch angle median.

Therefore, in an example implementation, which may be combined with any one or more of the embodiments and example implementations as described herein, the visual behavior monitor wearing status detection system is configured to determine improper wearing of the visual behavior monitor and/or the spectacles based on the pitch angle determined via the orientation sensor(s) in combination with distance data obtained via the distance sensor(s). Combining the data relating to the pitch angle obtained via the orientation sensor(s) with the distance data obtained via the distance sensor(s) to take into account the usage conditions of the visual behavior monitor and/or the spectacles may allow for a precise determination of the wearing status (proper or improper). This may in particular involve observation statistics to make this determination. In some example implementations, improper wearing of the visual behavior monitor and/or the spectacles may be determined if a measured pitch occurs more often and/or for a longer period of time (for example measured in percentage over a predetermined period of time) than expected, wherein the expected pitch is based on statistical data which may, in some examples, take into account the viewing distance(s) (e.g. far, intermediate, near). Taking into account the viewing distance(s) may be based on an occurrence of a certain pitch for far (e.g. above 2 meters), intermediate (e.g. between 0.5 meters and 2 meters) and near (e.g. below 0.5 meters) distances. The statistical data may hereby refer to a distribution of an (relative) occurrence as a function of the pitch angle, in particular based on a deviation from the horizon, and/or in particular based on a viewing distance.

Combining the sensor data obtained from the first and second sensors to determine whether the user is wearing the visual behaviour monitor or not may hereby be based on determining a combined probability for the user to wear the visual behaviour monitor, whereby individual probabilities for the user to wear the visual behaviour monitor from different sensors are combined to obtain the combined probability. This may advantageously allow for a more precise determination as to whether the user wears the visual behaviour monitor. For example, correct wearing of the monitor implies that the VBM is not upside down, it identifies attachment, proximity sensor senses presence of the object (head).

In another example, while the motion and attachment sensors may show that the visual behavior monitor is worn by the user, the orientation sensor may indicate that the visual behavior monitor is not worn. This may be an indication of, for example, the orientation sensor being worn upside down, such that the user should be alarmed (using, for example, the signal generator outlined above).

Combining data obtained from different sensors may therefore allow for improved determination as to whether the visual behavior monitor is worn by the user, and may in particular allow for warning the user if one or more of the sensors are not worn appropriately.

In a related aspect according to the present disclosure, there is provided a system comprising a visual behavior monitor for monitoring a visual behavior of a user, and the visual behavior monitor wearing status detection system, according to any one or more of the examples as described herein, coupled or integral to the visual behavior monitor.

### Brief Description of the Drawings

These and other aspects of the present disclosure will now be further described, by way of example only, with reference to the accompanying figures, wherein like reference numerals refer to like parts throughout, and in which:
- Figure 1: shows a schematic illustration of a visual behavior monitor wearing status detection system attached to spectacles, according to some example implementations as described herein;
- Figures 2a and 2b: show schematic illustrations of a use case of the visual behavior monitor wearing status detection system according to some example implementations as described herein;
- Figures 3a and 3b: show schematic illustrations of a further use case of the visual behavior monitor wearing status detection system according to some example implementations as described herein;
- Figures 4a and 4b: show schematic illustrations of a further use case of the visual behavior monitor wearing status detection system according to some example implementations as described herein;
- Figure 5: shows a schematic illustration of a further use case of the visual behavior monitor wearing status detection system according to some example implementations as described herein;
- Figure 6: shows a schematic illustration of a further use case of the visual behavior monitor wearing status detection system according to some example implementations as described herein;
- Figure 7: shows a schematic illustration of a further use case of the visual behavior monitor wearing status detection system according to some example implementations as described herein; and
- Figure 8: shows distribution of wearable pitch angles corresponding to near, intermediate and far viewing distances with the correct wearable attachment.

### Detailed Description

The present disclosure provides for a visual behavior monitor wearing status detection system which may be based on one or more clues (i.e. data) from one or more sensors in order to automatically recognise/determine that a visual behavior monitor is worn by the user or not.

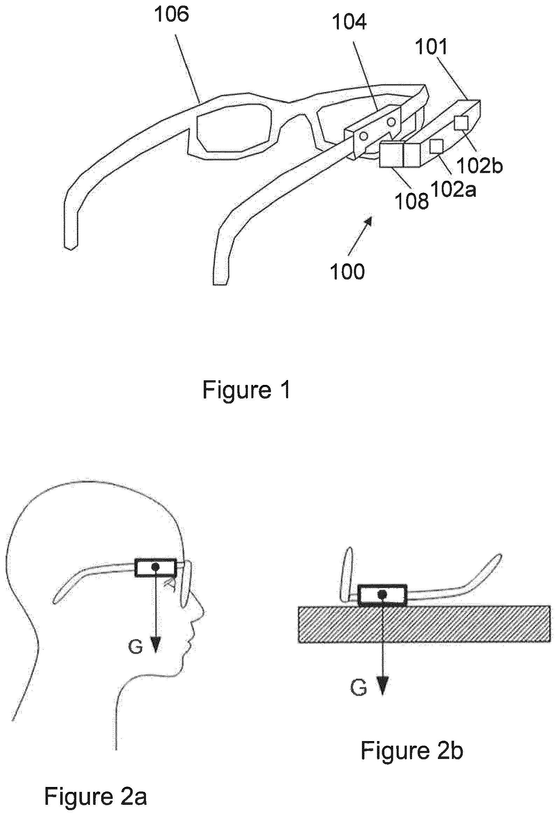

Figure 1 shows a schematic illustration of a visual behavior monitor wearing status detection system 101, according to some example implementations as described herein, which is attached, in this example, to spectacles 106.

The system 100 comprises, in this example, the visual behavior monitor wearing status detection system 101 and a visual behavior monitor 108. In this example, the visual behavior monitor wearing status detection system comprises a first sensor 102a and a second sensor 102b, which may be sensors as outlined above. A combination of different types of sensors may be provided.

In this example, the visual behavior monitor wearing status detection system 101 and the visual behavior monitor 108 are attached to an adapter or mount 104.

The attachment to the mount 104 may be used as a sensing means to sense whether the visual behavior monitor wearing status detection system 101 is attached to the mount 104, and hence the spectacles 106. An attachment sensor may hereby be comprised in the visual behavior monitor wearing status detection system 101. When the visual behavior monitor wearing status detection system 101 is not attached to the spectacles 106 and/or the mount 104, it may be determined that the visual behavior monitor 108 is not worn or used by the user.

Figures 2a and 2b show schematic illustrations of a use case of the visual behavior monitor wearing status detection system according to some example implementations as described herein.

In Figure 2a, that visual behavior monitor and the visual behavior monitor wearing status detection system are worn by the user. In Figure 2b, the spectacles and hence the visual behavior monitor and the visual behavior monitor wearing status detection system rest on a desk.

The sensor is, in this example, an orientation sensor. Gravity acting on the orientation sensor is indicated via the arrow labelled "G". Using the orientation sensor, it may be determined that the visual behavior monitor wearing status detection system and hence the visual behavior monitor rest on a desk (in this example upside down), which orientation is not compatible with normal usage. It may therefore be determined that the visual behavior monitor wearing status detection system and hence the visual behavior monitor are not worn by the user.

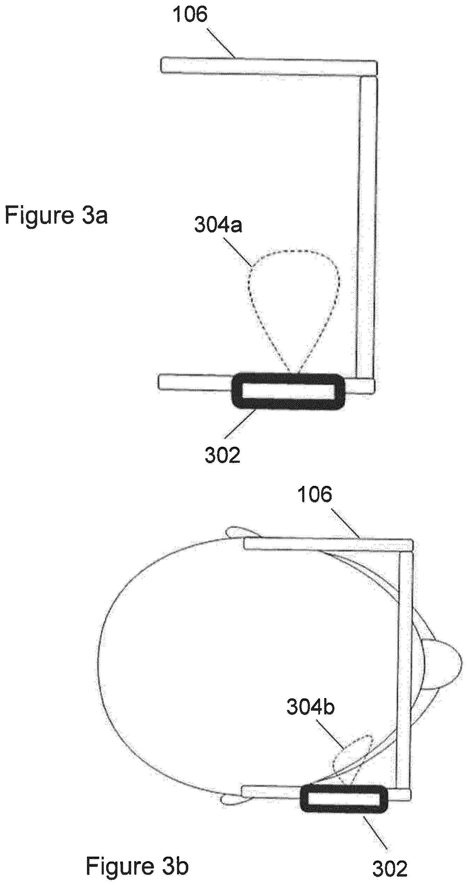

Figures 3a and 3b show schematic illustrations of a further use case of the visual behavior monitor wearing status detection system according to some example implementations as described herein.

In Figure 3a, the detection field 304a of the visual behavior monitor wearing status detection system, which comprises, in this example, a proximity sensor 302, is shown. As can be seen, when the spectacles 106 are not worn by the user, the detection field 304a extends, in this example, almost to the center of the spectacles, which is not possible under the normal usage.

In Figure 3b, the detection field 304b of the visual behavior monitor wearing status detection system, which comprises the proximity sensor 302, is shown for the case where the spectacles 106 are worn by the user. As can be seen, the detection field 304b when the user wears the spectacles 106 does not extend as far as the detection field 304a when the spectacles 106 are not worn by the user, since the signal is reflected by an object, in this example the user's head. The detection of wearing of the visual behavior monitor is performed using the proximity sensor 302 which is directed towards the head of the user. Based on the different distances (longer distance when the visual behavior monitor attached to the visual behavior monitor wearing status detection system is not worn by the user, compared to when the devices are worn), a determination may be made as to whether the user wears the visual behavior monitor or not.

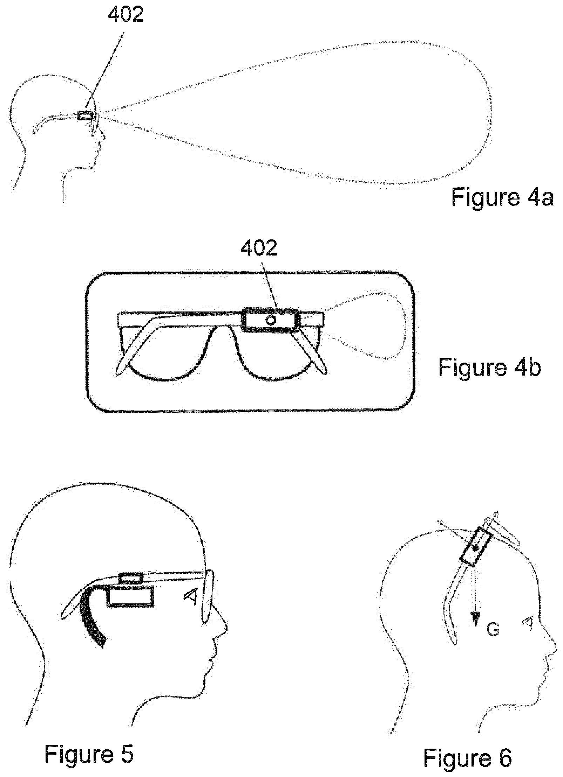

Figures 4a and 4b show schematic illustrations of a further use case of the visual behavior monitor wearing status detection system according to some example implementations as described herein.

The visual behavior monitor wearing status detection system shown in Figures 4a and 4b comprises, in this example, a distance sensor 402. In Figure 4a, while the visual behavior monitor wearing status detection system and hence the visual behavior monitor are worn by the user, the distance sensor 402 detects, in this example, a distance above a predetermined threshold distance, since under normal usage conditions, users do not face objects at relatively close distances. In the example shown in Figure 4b, the spectacles with the visual behavior monitor wearing status detection system comprising the distance sensor 402 are folded and put into a case/bag. The distance sensor 402 may therefore measure relatively short distances, which may serve as an indication of the devices not being used or worn by the user.

Figure 5 shows a schematic illustration of a further use case of the visual behavior monitor wearing status detection system according to some example implementations as described herein. In this example, the visual behavior monitor wearing status detection system is implemented as an earpiece which can be placed around the user's ear.

Figure 6 shows a schematic illustration of a further use case of the visual behavior monitor wearing status detection system according to some example implementations as described herein. In this scenario, the user wears spectacles, on which the visual behavior monitor wearing status detection system is arranged, on the top of his/her head. In such a scenario, when multiple sensors/clues/features are used in order to identify the wearing state/status, it may be possible to derive from the use of multiple sensors/clues/features that the spectacles/devices are worn, but in an incorrect manner. In this example, it may be determined that a distance sensor is covered/obstructed (for example by the user's hair or headgear), while the motion sensor may detect a motion of the devices and the orientation sensor may sense a deviation from a horizontal position of wearing the visual behavior monitor/spectacles. The combination of the information obtained via the different sensors may, in this example, allow for determining that the visual behavior monitor is worn, but potentially in an improper manner in view of the distance sensor being obstructed.

Detection of an improper usage of the device may be an important signal for visual behavior. In view of the above example, a patient may put spectacles on the top of his head or let spectacles hang on the chest, which may be an indication that the user is not using them currently, which information may be used in order to characterize the visual lifestyle of the user.

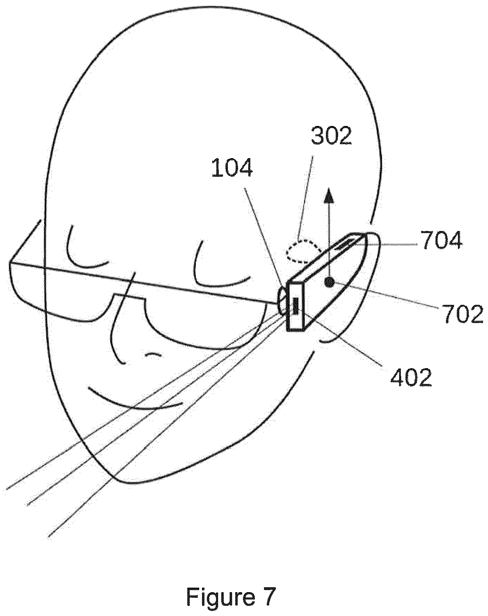

Figure 7 shows a schematic illustration of a further use case of the visual behavior monitor wearing status detection system according to some example implementations as described herein.

In this example, the visual behavior monitor wearing status detection system comprises a distance sensor 402 which is used to determine as to whether the distance to an object may be above or below a threshold distance.

In this example, the visual behavior monitor wearing status detection system further comprises a mount 104 via which it may be determined, for example, whether an attachment of the visual behavior monitor wearing status detection system to spectacles is present or not.

Furthermore, in this example, the visual behavior monitor wearing status detection system comprises an accelerometer 702 via which it may be determined as to whether the visual behavior monitor wearing status detection system is in an upright orientation or not.

Furthermore, in this example, the visual behavior monitor wearing status detection system comprises a proximity sensor 302 via which a proximity to the head of the user may be detected.

Furthermore, in this example, the visual behavior monitor wearing status detection system comprises an ambient light sensor 704 via which it may be determined as to whether sufficient illumination is present for the user to have vision, which may influence, for example, whether visual behaviour is being monitored or not depending on ambient light conditions.

In this example, the probability of wearing of the visual behavior monitor may be a multi-parameter model of statistical classification. All parameters may be taken into account at the same time in order to make a decision regarding the probability of wearing the visual behavior monitor.

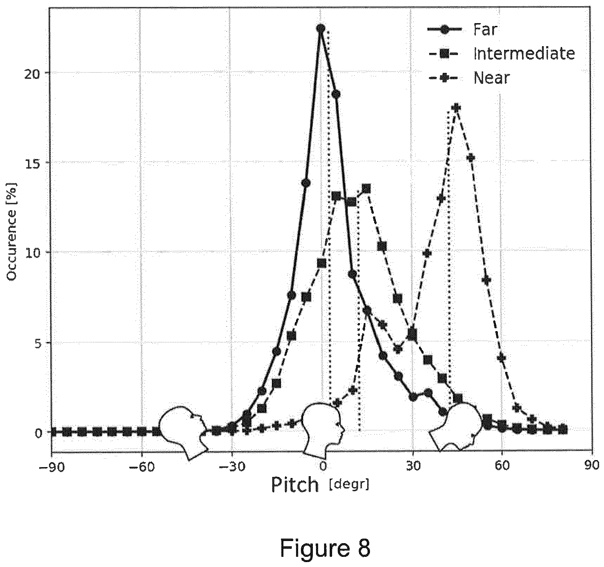

Figure 8 illustrates the distribution of wearable pitch angles which can be used to detect improper wearing and/or attachment of the wearable (visual behavior monitor and/or spectacles). In this example, the wearable is attached and used correctly, which results in a pitch corresponding to far distances (indicated with a solid thick curve) being distributed around zero horizontal level. The median of this distribution is below 5 degrees from horizon. In contrast, pitch distributions for intermediate and, especially, near distances are shifted downwards with medians above 10 and 40 degrees, respectively. Such pitch distribution for far distances is typical for normal usage with a proper attachment. Deviation from such statistics can be used to detect improper usage and/or attachment.

In some examples, an ambient light sensor may be used, which may be particularly advantageous in a case in which the visual behavior monitor wearing status detection system and hence the visual behavior monitor are in a closed case or bag. A relatively low light level (for example zero) below a predetermined threshold light level may be measured using the light sensor, while outside, during normal usage, the light level may be above the threshold light level (in order for the user to be able to use vision).

Each of the methods based on different sensors may detect signatures of the device not being used (for example, outside of normal usage), but such signatures may not be specific to the visual behavior monitor being used. For example, being attached to the adapter/mount may be required for proper use of the devices (necessity), this may not be sufficient (sufficiency), since a user may take off the spectacles with the mount and the visual behavior monitor/visual behavior monitor wearing status detection system and leave them on a desk, as shown, for example, in Figure 1.

Furthermore, while being in the proper vertical orientation (not being upside down) may be necessary for normal usage, the devices may be on the desk in the vertical orientation while not in use.

Furthermore, the devices experiencing some degree of motion may be necessary for normal usage, since a user may typically not be able to remain still (unless, for example, he/she sleeps). The device may also move while it is carried in a pocket.

Furthermore, devices measuring the presence of an object, such as, for example, the head of the user, with the proximity sensor may be necessary for normal usage, but may not be sufficient.

The above considerations may equally apply to measurements using the distance and ambient light sensors. Having a reasonable distance in front of the device and some level of ambient light may be necessary, but may not be sufficient.

It may therefore in some examples be preferable to combine data obtained from more than one sensor to more accurately determine as to whether the visual behavior monitor is worn by the user or not. This may be particularly advantageous so as to be able to implement statistical classification using machine learning techniques.

As outlined above, in some examples, the visual behavior monitor wearing status detection system may be part of an earpiece. As such, it may always be worn by the user and may, for example, be activated when the user puts on spectacles to which the visual behavior monitor wearing status detection system may be attached.

Throughout the present disclosure, any references to the "wearing status" of the visual behavior monitor may additionally or alternatively refer to the visual behavior monitor being used by the user or not. The phrase "wearing status" may therefore encompass one or both of the visual behavior monitor being worn and/or being used (in particular being used correctly or incorrectly) by the user.

No doubt many other effective alternatives will occur to the skilled person. It will be understood that the invention is not limited to the described embodiments and encompasses modifications apparent to those skilled in the art and lying within the scope of the claims appended hereto.

## Claims

1. A visual behavior monitor wearing status detection system (101) for detecting whether a visual behavior monitor (108), usable to monitor visual behavior of a user, is worn by the user, wherein the visual behavior monitor wearing status detection system (101) comprises:
sensors (102a, 102b) configured to obtain sensor data, and
wherein the visual behavior monitor wearing status detection system (101) is configured to process the sensor data to determine whether the visual behavior monitor is worn by the user;
**characterized by** the visual behavior monitor wearing status detection system (101) being further configured to combine the sensor data obtained from a first sensor (102a) and a second sensor (102b) of said sensors to statistically determine whether the user is correctly wearing the visual behavior monitor (108) or is wearing the visual behavior monitor (108) in a manner previously determined to be improper.

2. A visual behavior monitor wearing status detection system (101) as claimed in claim 1, wherein said determining, by the visual behavior monitor wearing status detection system (101), whether the visual behavior monitor (108) is worn by the user comprises automatically determining whether a predefined condition, derived by the visual behavior monitor wearing status detection system (101) from the sensor data, is fulfilled.

3. A visual behavior monitor wearing status detection system (101) as claimed in claim 1 or 2, wherein the determination whether the visual behavior monitor (108) is worn by the user comprises calculating, by the visual behavior monitor wearing status detection system (101), a probability for the user to wear the visual behavior monitor (108) and determining whether the probability exceeds or falls below a threshold.

4. A visual behavior monitor wearing status detection system (101) as claimed in any preceding claim, further comprising a controller for controlling, based on the determination whether the visual behavior monitor (108) is worn by the user, one or more of:
(i) starting and/or stopping of the visual behavior monitoring of the user via the visual behavior monitor (108), and
(ii) activating and/or deactivating components of the visual behavior monitor (108).

5. A visual behavior monitor wearing status detection system (101) as claimed in any preceding claim, wherein the sensors (102a, 102b) comprise one or more orientation sensors for obtaining orientation data relating to an orientation of the visual behavior monitor wearing status detection system (101), and wherein the sensor data, processed to determine whether the visual behavior monitor (108) is worn by the user, comprises said orientation data.

6. A visual behavior monitor wearing status detection system (101) as claimed in claim 5, wherein the visual behavior monitor wearing status detection system (101) is configured to determine, from the orientation data, one or both of a pitch and a roll of the visual behavior monitor wearing status detection system, and wherein the determination whether the visual behavior monitor (108) is worn by the user is based on the pitch and/or the roll of the visual behavior monitor wearing status detection system (101) satisfying an orientation condition.

7. A visual behavior monitor wearing status detection system (101) as claimed in claim 5 or 6, wherein the one or more orientation sensors comprise a first accelerometer (702).

8. A visual behavior monitor wearing status detection system as claimed in any preceding claim, wherein the sensors (102a, 102b) comprise one or more motion sensors for obtaining motion data relating to a type and/or an amount of motion of the visual behavior monitor wearing status detection system (101), and wherein the sensor data, processed to determine whether the visual behavior monitor (108) is worn by the user, comprises said motion data.

9. A visual behavior monitor wearing status detection system as claimed in claim 8, when dependent on claim 4, wherein the visual behavior monitor wearing status detection system (101) is configured to stop and/or prevent starting the visual behavior monitoring of the user via the visual behavior monitor (108) and/or activate and/or deactivate components of the visual behavior monitor (108) when a first time period, for which the amount of motion of the visual behavior monitor wearing status detection system (101) falls outside of a predefined range, exceeds a first threshold time period.

10. A visual behavior monitor wearing status detection system (101) as claimed in claim 8 or 9, wherein the one or more motion sensors comprise one or more of: a gyroscope, a magnetic sensor, a magnetometer, an inertial sensor and a second accelerometer, in particular wherein the second accelerometer is integral to the first accelerometer of claim 7.

11. A visual behavior monitor wearing status detection system (101) as claimed in any preceding claim, wherein the sensors (102a, 102b) comprise one or more distance sensors (402) for obtaining distance data relating to a distance between the visual behavior monitor wearing status detection system (101) and an object, wherein the sensor data, processed to determine whether the visual behavior monitor (108) is worn by the user, comprises said distance data, and wherein the visual behavior monitor wearing status detection system (101) is configured to determine that the visual behavior monitor (108) is not worn by the user when the distance is below a threshold distance, in particular when the distance is below the threshold distance for a second time period exceeding a second threshold time period, and/or;
wherein the sensors (102a, 102b) comprise one or more proximity sensors (302) for obtaining proximity data relating to a proximity of the visual behavior monitor wearing status detection system (101) to a body part of the user, wherein the sensor data, processed to determine whether the visual behavior monitor (108) is worn by the user, comprises said proximity data, and wherein the visual behavior monitor wearing status detection system (101) is configured to determine that the visual behavior monitor (108) is worn by the user when the proximity is below a threshold proximity.

12. A visual behavior monitor wearing status detection system (101) as claimed in any preceding claim, wherein the sensors (102a, 102b) comprise one or more light sensors (704) for obtaining light intensity data relating to a light intensity of ambient light impinging on the one or more light sensors (704),
wherein the sensor data, processed to determine whether the visual behavior monitor is worn by the user, comprises said light intensity data, and
wherein the visual behavior monitor wearing status detection system (101) is configured to determine that the visual behavior monitor (108) is not worn by the user when the light intensity is below a threshold light intensity, in particular
wherein the visual behavior monitor wearing status detection system (101) is configured to determine that the visual behavior monitor (108) is not worn by the user when the light intensity is below the threshold light intensity for longer than a third threshold time period.

13. A visual behavior monitor wearing status detection system (101) as claimed in any preceding claim, wherein the sensors (102a, 102b) comprise one or more attachment sensors for obtaining attachment data relating to an attachment of one or both of the visual behavior monitor wearing status detection system (101) and the visual behavior monitor (108) to spectacles and/or an ear or another part of the user's head and/or another fixation,
wherein the sensor data, processed to determine whether the visual behavior monitor is worn by the user, comprises said attachment data, and
wherein the visual behavior monitor wearing status detection system (101) is configured to determine that the visual behavior monitor (108) is worn by the user when it is determined that one or both of the visual behavior monitor wearing status detection system (101) and the visual behavior monitor (108), respectively, are attached to the spectacles and/or the ear and/or the another part of the user's head and/or the fixation.

14. A system (101) comprising:
a visual behavior monitor (108) for monitoring a visual behavior of a user, and the visual behavior monitor wearing status detection system (101) of any one of the preceding claims coupled or integral to the visual behavior monitor (108).

## Patentansprüche

1. Ein Tragezustandserfassungssystem (101) für ein Sehverhaltensüberwachungsgerät zum Erfassen, ob ein Sehverhaltensüberwachungsgerät (108), das zum Überwachen eines Sehverhaltens eines Benutzers verwendbar ist, von dem Benutzer getragen wird, wobei das Tragezustandserfassungssystem (101) für ein Sehverhaltensüberwachungsgerät umfasst:
Sensoren (102a, 102b), die konfiguriert sind, Sensordaten zu erhalten, und
wobei das Tragezustandserfassungssystem (101) für ein Sehverhaltensüberwachungsgerät konfiguriert ist, die Sensordaten zu verarbeiten, um zu bestimmen, ob das Sehverhaltensüberwachungsgerät von dem Benutzer getragen wird;
**gekennzeichnet dadurch,**
**dass** das Tragezustandserfassungssystem (101) für ein Sehverhaltensüberwachungsgerät ferner konfiguriert ist, die von einem ersten Sensor (102a) und einem zweiten Sensor (102b) der Sensoren erhaltenen Sensordaten zu kombinieren, um statistisch zu bestimmen, ob der Benutzer das Sehverhaltensüberwachungsgerät (108) korrekt trägt oder das Sehverhaltensüberwachungsgerät (108) in einer zuvor als unpassend bestimmten Weise trägt.

2. Ein Tragezustandserfassungssystem (101) für ein Sehverhaltensüberwachungsgerät nach Anspruch 1, wobei das Bestimmen, durch das Tragezustandserfassungssystem (101) für ein Sehverhaltensüberwachungsgerät, ob das Sehverhaltensüberwachungsgerät (108) von dem Benutzer getragen wird, ein automatisches Bestimmen umfasst, ob eine vordefinierte Bedingung, die durch das Tragezustandserfassungssystem (101) für ein Sehverhaltensüberwachungsgerät aus den Sensordaten abgeleitet wurde, erfüllt ist.

3. Ein Tragezustandserfassungssystem (101) für ein Sehverhaltensüberwachungsgerät nach Anspruch 1 oder 2, wobei das Bestimmen, durch das Tragezustandserfassungssystem (101) für ein Sehverhaltensüberwachungsgerät, ob das Sehverhaltensüberwachungsgerät (108) von dem Benutzer getragen wird, ein Berechnen einer Wahrscheinlichkeit für den Benutzer, das Sehverhaltensüberwachungsgerät (108) zu tragen, und ein Bestimmen umfasst, ob die Wahrscheinlichkeit einen Schwellenwert überschreitet oder unterschreitet.

4. Ein Tragezustandserfassungssystem (101) für ein Sehverhaltensüberwachungsgerät nach einem der vorhergehenden Ansprüche, ferner umfassend eine Steuerung zum Steuern, basierend auf der Bestimmung, ob das Sehverhaltensüberwachungsgerät (108) von dem Benutzer getragen wird, eines oder mehrere von:
(i) Starten und/oder Stoppen der Sehverhaltensüberwachung des Benutzers über das Sehverhaltensüberwachungsgerät (108), und
(ii) Aktivieren und/oder Deaktivieren von Komponenten des Sehverhaltensüberwachungsgeräts (108).

5. Ein Tragezustandserfassungssystem (101) für ein Sehverhaltensüberwachungsgerät nach einem der vorhergehenden Ansprüche, wobei die Sensoren (102a, 102b) einen oder mehrere Orientierungssensoren zum Erhalten von Orientierungsdaten umfassen, die sich auf eine Orientierung des Tragezustandserfassungssystems (101) für ein Sehverhaltensüberwachungsgerät beziehen, und wobei die Sensordaten, die verarbeitet werden, um zu bestimmen, ob das Sehverhaltensüberwachungsgerät (108) von dem Benutzer getragen wird, die Orientierungsdaten umfassen.

6. Ein Tragezustandserfassungssystem (101) für ein Sehverhaltensüberwachungsgerät nach Anspruch 5, wobei das Tragezustandserfassungssystem (101) für ein Sehverhaltensüberwachungsgerät konfiguriert ist, aus den Orientierungsdaten eine Neigung und/oder ein Rollen des Tragezustandserfassungssystems für ein Sehverhaltensüberwachungsgerät zu bestimmen, und wobei die Bestimmung, ob das Sehverhaltensüberwachungsgerät (108) von dem Benutzer getragen wird, auf der Neigung und/oder dem Rollen des Tragezustandserfassungssystems (101) für ein Sehverhaltensüberwachungsgerät basiert, die eine Orientierungsbedingung erfüllen.

7. Ein Tragezustandserfassungssystem (101) für ein Sehverhaltensüberwachungsgerät nach Anspruch 5 oder 6, wobei der eine oder die mehreren Orientierungssensoren einen ersten Beschleunigungsmesser (702) umfassen.

8. Ein Tragezustandserfassungssystem (101) für ein Sehverhaltensüberwachungsgerät nach einem der vorhergehenden Ansprüche, wobei die Sensoren (102a, 102b) einen oder mehrere Bewegungssensoren zum Erhalten von Bewegungsdaten umfassen, die sich auf einen Typ und/oder einen Betrag einer Bewegung des Tragezustandserfassungssystems (101) für ein Sehverhaltensüberwachungsgerät beziehen, und wobei die Sensordaten, die verarbeitet werden, um zu bestimmen, ob das Sehverhaltensüberwachungsgerät (108) von dem Benutzer getragen wird, die Bewegungsdaten umfassen.

9. Ein Tragezustandserfassungssystem (101) für ein Sehverhaltensüberwachungsgerät nach Anspruch 8, wenn abhängig von Anspruch 4, wobei das Tragezustandserfassungssystem (101) für ein Sehverhaltensüberwachungsgerät konfiguriert ist, die Sehverhaltensüberwachung des Benutzers über das Sehverhaltensüberwachungsgerät (108) zu stoppen und/oder das Starten zu verhindern und/oder Komponenten des Sehverhaltensüberwachungsgeräts (108) zu aktivieren und/oder zu deaktivieren, wenn eine erste Zeitspanne, für die der Betrag der Bewegung des Tragezustandserfassungssystems (101) für ein Sehverhaltensüberwachungsgerät außerhalb eines vordefinierten Bereichs liegt, eine erste Schwellenzeitspanne überschreitet.

10. Ein Tragezustandserfassungssystem (101) für ein Sehverhaltensüberwachungsgerät nach Anspruch 8 oder 9, wobei die einen oder mehreren Bewegungssensoren einen oder mehrere umfassen von: einem Gyroskop, einem Magnetsensor, einem Magnetometer, einem Trägheitssensor und einem zweiten Beschleunigungsmesser, insbesondere wobei der zweite Beschleunigungsmesser in dem ersten Beschleunigungsmesser nach Anspruch 7 integriert ist.

11. Ein Tragezustandserfassungssystem (101) für ein Sehverhaltensüberwachungsgerät nach einem der vorhergehenden Ansprüche, wobei die Sensoren (102a, 102b) einen oder mehrere Abstandssensoren (402) zum Erhalten von Abstandsdaten umfassen, die sich auf einen Abstand zwischen dem Tragezustandserfassungssystem (101) für ein Sehverhaltensüberwachungsgerät und einem Objekt beziehen, wobei die Sensordaten, die verarbeitet werden, um zu bestimmen, ob das Sehverhaltensüberwachungsgerät (108) von dem Benutzer getragen wird, die Abstandsdaten umfasst, und wobei das Tragezustandserfassungssystem (101) für ein Sehverhaltensüberwachungsgerät konfiguriert ist, zu bestimmen, dass das Sehverhaltensüberwachungsgerät (108) von dem Benutzer nicht getragen wird, wenn der Abstand unterhalb eines Schwellenabstands liegt, insbesondere wenn der Abstand für eine zweite Zeitspanne, die eine zweite Schwellenzeitspanne überschreitet, unterhalb des Schwellenabstands liegt, und/oder;
wobei die Sensoren (102a, 102b) einen oder mehrere Näherungssensoren (302) zum Erhalten von Näherungsdaten umfassen, die sich auf eine Nähe des Tragezustandserfassungssystems (101) für ein Sehverhaltensüberwachungsgerät zu einem Körperteil des Benutzers beziehen, wobei die Sensordaten, die verarbeitet werden, um zu bestimmen, ob das Sehverhaltensüberwachungsgerät (108) von dem Benutzer getragen wird, die Näherungsdaten umfassen, und wobei das Ein Tragezustandserfassungssystem (101) für ein Sehverhaltensüberwachungsgerät konfiguriert ist, zu bestimmen, dass das Sehverhaltensüberwachungsgerät (108) von dem Benutzer getragen wird, wenn die Nähe unter einem Nähe-Schwellenwert liegt.

12. Ein Tragezustandserfassungssystem (101) für ein Sehverhaltensüberwachungsgerät nach einem der vorhergehenden Ansprüche, wobei die Sensoren (102a, 102b) einen oder mehrere Lichtsensoren (704) zum Erhalten von Lichtintensitätsdaten umfassen, die sich auf eine Lichtintensität von Umgebungslicht beziehen, das auf den einen oder die mehreren Lichtsensoren (704) auftrifft,
wobei die Sensordaten, die verarbeitet werden, um zu bestimmen, ob das Sehverhaltensüberwachungsgerät (108) von dem Benutzer getragen wird, die Lichtintensitätsdaten umfassen, und
wobei das Tragezustandserfassungssystem (101) für ein Sehverhaltensüberwachungsgerät konfiguriert ist, zu bestimmen, dass das Sehverhaltensüberwachungsgerät (108) von dem Benutzer nicht getragen wird, wenn die Lichtintensität unter einem Schwellenwert der Lichtintensität liegt, insbesondere
wobei das Tragezustandserfassungssystem (101) für ein Sehverhaltensüberwachungsgerät konfiguriert ist, zu bestimmen, dass das Sehverhaltensüberwachungsgerät (108) von dem Benutzer nicht getragen wird, wenn die Lichtintensität länger als eine dritte Schwellenwertzeitspanne unter der Schwellenlichtintensität liegt.

13. Ein Tragezustandserfassungssystem (101) für ein Sehverhaltensüberwachungsgerät nach einem der vorhergehenden Ansprüche, wobei die Sensoren (102a, 102b) einen oder mehrere Anbringungssensoren zum Erhalten von Anbringungsdaten umfassen, die sich auf eine Anbringung eines oder beider des Tragezustandserfassungssystems (101) für ein Sehverhaltensüberwachungsgerät und des Sehverhaltensüberwachungsgeräts (108) an einer Brille und/oder einem Ohr oder einem anderen Teil des Kopfes des Benutzers und/oder einer anderen Fixierung beziehen,
wobei die Sensordaten, die verarbeitet werden, um zu bestimmen, ob das Sehverhaltensüberwachungsgerät (108) von dem Benutzer getragen wird, die genannten Anbringungsdaten umfassen, und
wobei das Tragezustandserfassungssystem (101) für ein Sehverhaltensüberwachungsgerät konfiguriert ist, zu bestimmen, dass das Sehverhaltensüberwachungsgerät (108) von dem Benutzer getragen wird, wenn bestimmt wird, dass eines oder beide des Tragezustandserfassungssystems (101) für ein Sehverhaltensüberwachungsgerät und des Sehverhaltensüberwachungsgeräts (108) jeweils an der Brille und/oder dem Ohr und/oder dem anderen Teil des Kopfes des Benutzers und/oder der Fixierung angebracht sind.

14. Ein System (101) umfassend:
ein Sehverhaltensüberwachungsgerät (108) zur Überwachung eines Sehverhaltens eines Benutzers, und
das Tragezustandserfassungssystem (101) für ein Sehverhaltensüberwachungsgerät nach einem der vorhergehenden Ansprüche, das mit dem Sehverhaltensüberwachungsgerät (108) gekoppelt oder in dieses integriert ist.

## Revendications

1. Système de détection (101) d'état d'utilisation d'un moniteur de comportement visuel pour détecter si un moniteur de comportement visuel (108), servant à surveiller le comportement visuel d'un utilisateur, est porté par l'utilisateur, le système de détection (101) d'état d'utilisation d'un moniteur de comportement visuel comprenant :
des capteurs (102a, 102b) conçus pour obtenir des données de capteur, et
le système de détection (101) d'état d'utilisation d'un moniteur de comportement visuel étant conçu pour traiter les données de capteur pour déterminer si le moniteur de comportement visuel est porté par l'utilisateur ;
**caractérisé par**
le système de détection (101) d'état d'utilisation d'un moniteur de comportement visuel est en outre conçu pour combiner les données de capteur obtenues à partir d'un premier capteur (102a) et d'un second capteur (102b) desdits capteurs pour déterminer en termes statistiques si l'utilisateur porte correctement le moniteur de comportement visuel (108) ou porte le moniteur de comportement visuel (108) d'une manière déterminée précédemment comme inadaptée.

2. Système de détection (101) d'état d'utilisation d'un moniteur de comportement visuel selon la revendication 1, ladite détermination, par le système de détection (101) d'état d'utilisation d'un moniteur de comportement visuel, si le moniteur de comportement visuel (108) est porté par l'utilisateur consistant à déterminer automatiquement si une condition prédéfinie, calculée par le système de détection (101) d'état d'utilisation d'un moniteur de comportement visuel, provenant des données de capteur, est remplie.

3. Système de détection (101) d'état d'utilisation d'un moniteur de comportement visuel selon la revendication 1 ou la revendication 2, la détermination si le moniteur de comportement visuel (108) est porté par l'utilisateur, consistant à calculer, par le système de détection (101) d'état d'utilisation d'un moniteur de comportement visuel, une probabilité pour l'utilisateur de porter le moniteur de comportement visuel (108) et déterminer si la probabilité est supérieure ou inférieure à un seuil.

4. Système de détection (101) d'état d'utilisation d'un moniteur de comportement visuel selon l'une quelconque revendication précédente, comprenant en outre un organe de commande pour commander, en fonction de la détermination si le moniteur de comportement visuel (108) est porté par l'utilisateur, une ou plusieurs actions :
(i) démarrer et/ou arrêter la surveillance du comportement visuel de l'utilisateur par l'intermédiaire du moniteur de comportement visuel (108), et
(ii) activer et/ou désactiver les composants du moniteur de comportement visuel (108).

5. Système de détection (101) d'état d'utilisation d'un moniteur de comportement visuel selon l'une quelconque revendication précédente, les capteurs (102, 102b) comprenant un ou plusieurs capteurs d'orientation pour obtenir des données d'orientation se rapportant à une orientation du système de détection (101) d'état d'utilisation d'un moniteur de comportement visuel, et les données de capteur, traitées pour déterminer si le moniteur de comportement visuel (108) est porté par l'utilisateur, comprend lesdites données d'orientation.

6. Système de détection (101) d'état d'utilisation d'un moniteur de comportement visuel selon la revendication 5, le système de détection (101) d'état d'utilisation d'un moniteur de comportement visuel étant conçu pour déterminer, à partir des données d'orientation, un tangage et/ou un roulis du système de détection (101) d'état d'utilisation d'un moniteur de comportement visuel, et la détermination si le moniteur de comportement visuel (108) est porté par l'utilisateur étant basée sur le tangage et/ou le roulis du système de détection (101) d'état d'utilisation d'un moniteur de comportement visuel remplissant une condition d'orientation.

7. Système de détection (101) d'état d'utilisation d'un moniteur de comportement visuel selon la revendication 5 ou la revendication 6, lesdits capteurs d'orientation comprenant un premier accéléromètre (702).

8. Système de détection (101) d'état d'utilisation d'un moniteur de comportement visuel selon l'une quelconque revendication précédente, les capteurs (102a, 102b) comprenant un ou plusieurs capteurs de mouvement pour obtenir des données de mouvement se rapportant à un type et/ou à une quantité de mouvement du système de détection (101) d'état d'utilisation d'un moniteur de comportement visuel, et les données de capteurs, traitées pour déterminer si le moniteur de comportement visuel (108) est porté par l'utilisateur, comprenant lesdites données de mouvement.

9. Système de détection (101) d'état d'utilisation d'un moniteur de comportement visuel selon la revendication 8, lorsqu'il relève de la revendication 4, le système de détection (101) d'état d'utilisation d'un moniteur de comportement visuel étant conçu pour arrêter et/ou empêcher le démarrage de la surveillance de comportement visuel de l'utilisateur par l'intermédiaire du moniteur de comportement visuel (108) et/ou activer et/ou désactiver les composants du moniteur de comportement visuel (108) lorsqu'une première fois la quantité de mouvement du système de détection (101) d'état d'utilisation d'un moniteur de comportement visuel se trouve en dehors d'une plage prédéfinie, dépasse une première période de temps seuil.

10. Système de détection (101) d'état d'utilisation d'un moniteur de comportement visuel selon la revendication 8 ou la revendication 9, lesdits capteurs comprenant un ou plusieurs éléments suivants : un gyroscope et/ou un capteur magnétique et/ou un magnétomètre et/ou un capteur d'inertie et/ou un second accéléromètre, en particulier le second accéléromètre faisant partie intégrante du premier accéléromètre selon la revendication 7.

11. Système de détection (101) d'état d'utilisation d'un moniteur de comportement visuel selon l'une quelconque revendication précédente, les capteurs (102a, 102b) comprenant un ou plusieurs capteurs de distance (402) pour obtenir des données de distance se rapportant à une distance entre le système de détection (101) d'état d'utilisation d'un moniteur de comportement visuel et un objet, les données de capteur traitées pour déterminer si le moniteur de comportement visuel (108) est porté par l'utilisateur, comprenant lesdites données de distance et le système de détection (101) d'état d'utilisation d'un moniteur de comportement visuel étant conçu pour déterminer que le moniteur de comportement visuel (108) n'est pas porté par l'utilisateur lorsque la distance est inférieure à une distance seuil, en particulier lorsque la distance est inférieure à la distance seuil pour une seconde période de temps dépassant une seconde période de temps seuil, et/ou ;
les capteurs (102a, 102b) comprenant un ou plusieurs capteurs de proximité (302) pour obtenir des données de proximité se rapportant à une proximité entre le système de détection (101) d'état d'utilisation d'un moniteur de comportement visuel et une partie du corps de l'utilisateur, les données de capteurs, traitées pour déterminer si le moniteur de comportement visuel (108) est porté par l'utilisateur, comprenant lesdites données de proximité, et le système de détection (101) d'état d'utilisation d'un moniteur de comportement visuel étant conçu pour déterminer que le moniteur de comportement visuel (108) est porté par l'utilisateur lorsque la proximité est inférieure à une proximité seuil.

12. Système de détection (101) d'état d'utilisation d'un moniteur de comportement visuel selon l'une quelconque revendication précédente, les capteurs (102a, 102b) comprenant un ou plusieurs capteurs de lumière (704) pour obtenir des données d'intensité lumineuse se rapportant à une intensité lumineuse de la lumière ambiante ayant un impact sur lesdits capteurs de lumière (704),
les données de capteur, traitées pour déterminer su le moniteur de comportement visuel est porté par l'utilisateur, comprenant lesdites données d'intensité lumineuse, et
le système de détection (101) d'état d'utilisation d'un moniteur de comportement visuel étant conçu pour déterminer que le moniteur de comportement visuel (108) n'est pas porté par l'utilisateur lorsque l'intensité lumineuse est inférieure à une intensité lumineuse seuil, en particulier
le système de détection (101) d'état d'utilisation d'un moniteur de comportement visuel étant conçu pour déterminer que le moniteur de comportement visuel (108) n'est pas porté par l'utilisateur lorsque l'intensité lumineuse est inférieure à l'intensité lumineuse seuil pendant plus longtemps qu'une troisième période de temps seuil.

13. Système de détection (101) d'état d'utilisation d'un moniteur de comportement visuel selon l'une quelconque revendication précédente, les capteurs (102a, 102b) comprenant un ou plusieurs capteurs de fixation pour obtenir des données de fixation se rapportant à une fixation du système de détection (101) d'état d'utilisation d'un moniteur de comportement visuel et/ou du moniteur de comportement visuel (108) à des lunettes et/ou à une oreille ou une autre partie de la tête de l'utilisateur et/ou à une autre fixation,
les données de capteur, traitées pour déterminer si le moniteur de comportement visuel est porté par l'utilisateur, comprenant lesdites données de fixation, et
le système de détection (101) d'état d'utilisation d'un moniteur de comportement visuel étant conçu pour déterminer que le moniteur de comportement visuel (101) est porté par l'utilisateur lorsqu'il est déterminé que le système de détection (101) d'état d'utilisation d'un moniteur de comportement visuel et/ou le moniteur de comportement visuel (108), respectivement, sont fixés aux lunettes et/ou à l'oreille et/ou à une autre partie de la tête de l'utilisateur et/ou à une fixation.

14. Système (101) comprenant :
un moniteur de comportement visuel (108) pour surveiller un comportement visuel d'un utilisateur, et le système de détection (101) d'état d'utilisation d'un moniteur de comportement visuel selon l'une quelconque des revendications précédentes couplées au moniteur de comportement visuel (108) ou faisant partie intégrante de celui-ci.
